Europäisches Patentamt

European Patent Office

Office européen des brevets

(11) Veröffentlichungsnummer: **0 277 489**

**A2**

(12) # EUROPÄISCHE PATENTANMELDUNG

(21) Anmeldenummer: 88100261.2

(51) Int. Cl.⁴: **A61B 17/22**

(22) Anmeldetag: 11.01.88

(30) Priorität: 26.01.87 DE 8701218 U

(43) Veröffentlichungstag der Anmeldung:
10.08.88 Patentblatt 88/32

(84) Benannte Vertragsstaaten:
**DE FR GB NL**

(71) Anmelder: **Siemens Aktiengesellschaft Berlin
und München
Wittelsbacherplatz 2
D-8000 München 2(DE)**

(72) Erfinder: **Noske, Erich
Ruhsteinweg 12
D-8525 Weiher(DE)**
Erfinder: **Reichenberger, Helmut, Dr.
Begonienstrasse 28
D-8501 Eckental(DE)**
Erfinder: **Pfeiler, Manfred, Dr.
Ludwig-Thoma-Strasse 25
D-8520 Erlangen(DE)**

(54) **Lithotripsie-Arbeitsplatz.**

(57) Die Erfindung betrifft einen Lithotripsie-Arbeitsplatz mit mehreren, an einer Halterung (7, 8, 9) einstellbar gelagerten Stoßwellengeneratoren (3, 4).

Bei dem erfindungsgemäßen Arbeitsplatz ist die Beschallung von Konkrementen (2) mit Stoßwellen unter Winkeln möglich, die der jeweiligen klinischen Situation variabel anpaßbar sind. Hierzu ist die Halterung (7, 8, 9) so ausgebildet, daß die Stoßwellengeneratoren (3, 4) einzeln unabhängig voneinander einstellbar sind.

EP 0 277 489 A2

## Lithotripsie-Arbeitsplatz

Die Erfindung betrifft einen Lithotripsie-Arbeitsplatz mit mehreren, an einer Halterung einstellbar gelagerten, gleichzeitig am Patienten applizierbaren Stoßwellengeneratoren.

Es ist ein Lithotripsie-Arbeitsplatz bekannt, bei dem die Stoßwellengeneratoren unter einer Patientenliege derart gelagert sind, daß jeweils einer aus einer Parkstellung in eine Arbeitsstellung - schwenkbar ist, in der er mit seiner Applikationsseite am Patienten anlegbar ist. Der Winkel, unter dem die Stoßwellengeneratoren an einem gemeinsamen Halter angeordnet sind, ist dabei fest.

Der Erfindung liegt die Aufgabe zugrunde, einen Lithotripsie-Arbeitsplatz der eingangs genannten Art so auszubilden, daß die Beschallung von Konkrementen mit Stoßwellen unter der jeweiligen klinischen Situation variabel anpaßbaren Winkeln möglich ist.

Diese Aufgabe ist erfindungsgemäß dadurch gelöst, daß die Halterung so ausgebildet ist, daß der Einstrahlwinkel für jeden Stoßwellengenerator individuell einstellbar ist. Bei dem erfindungsgemäßen Lithotripsie-Arbeitsplatz kann jeder Stoßwellengenerator innerhalb eines großen Bereiches individuell auf das jeweils zu beschallende Konkrement eingestellt werden, so daß der jeweiligen klinischen Situation optimal Rechnung getragen werden kann. Es ist dabei möglich, zwei oder mehr Stoßwellengeneratoren an der Halterung individuell zu haltern. Die Stoßwellengeneratoren können dabei beliebig ausgebildet sein, z.B. vom elektromagnetischen oder piezokeramischen Typ sein. Im Betrieb kann jeder Stoßwellengenerator individuell über ein Kontaktmittel, z.B. eine durch Flüssigkeitsdruck aufblasbare Membran an den Patientenkörper angekoppelt werden.

Eine zweckmäßige konstruktive Lösung besteht darin, daß die Halterung eine etwa kreisförmig gekrümmte Führung für die Stoßwellengeneratoren aufweist, die mittels eines Kugelgelenkes allseitig - schwenkbar gelagert ist. Bei dieser Ausführung ist eine besonders vielseitige Einstellung jedes einzelnen Stoßwellengenerators im Raum möglich.

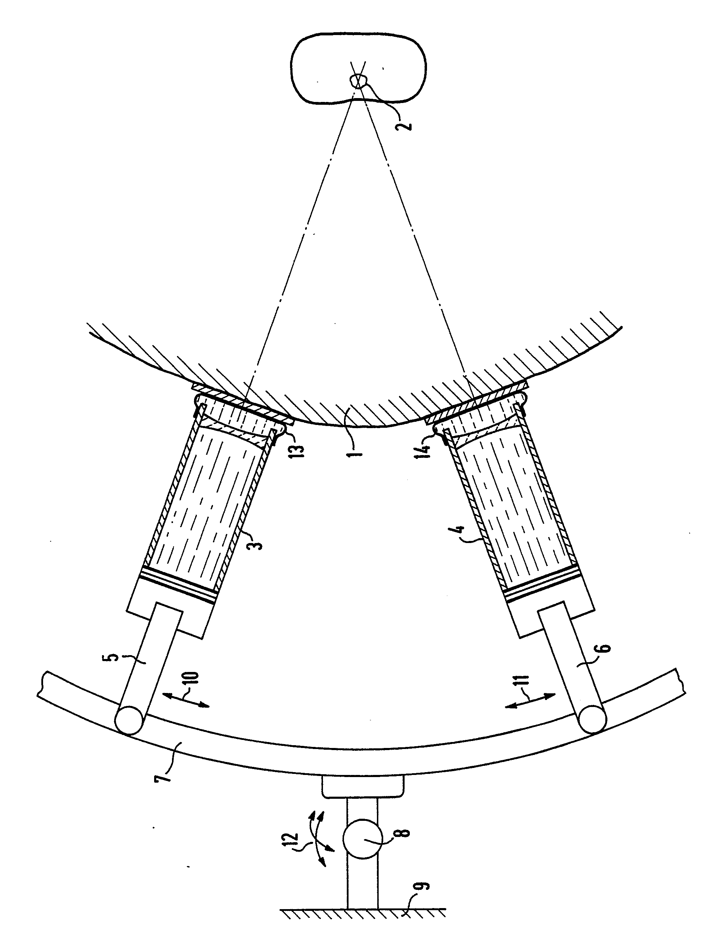

Die Erfindung ist nachfolgend anhand eines in der Zeichnung dargestellten Ausführungsbeispieles näher erläutert.

In der Zeichnung ist ein Patient 1 dargestellt, der bei dem Beispiel einen Nierenstein 2 hat. Der Nierenstein 2 soll durch Beschallung mit von Stoßwellengeneratoren 3, 4 ausgehenden Stoßwellen zertrümmert werden. Die Stoßwellengeneratoren 3, 4 sind dabei in bekannter Weise ausgebildet (s. z.B. die DE-OS 33 28 051). Jeder Stoßwellengenerator 3, 4 ist mittels eines Armes 5, 6 an einer kreisförmig gekrümmten Schiene 7 längsverschiebbar und feststellbar gelagert. Die Schiene 7 ist über ein Kugelgelenk 8 mit einem gerätefesten Gestell 9 verbunden. Demgemäß sind die Stoßwellengeneratoren 3, 4 gegenüber der Schiene 7 in Richtung der Doppelpfeile 10, 11 verstellbar und die Einheit 3, 4, 7 ist mit Hilfe des Kugelgelenkes 8 in Richtung der Pfeile 12 im Raum einstellbar und feststellbar.

Derjenige Stoßwellengenerator 3, 4, mit dem jeweils der Nierenstein 2 beschallt werden soll, wird an der Oberfläche des Patienten 1 mit Hilfe einer hydraulisch aufblasbaren Membran 13, 14 angelegt.

Je nach klinischer Situation können die Stoßwellengeneratoren 3, 4 einzeln oder gemeinsam betrieben (ausgelöst) werden.

### Ansprüche

1. Lithotripsie-Arbeitsplatz mit mehreren, an einer Halterung (7, 8, 9) einstellbar gelagerten, gleichzeitig am Patienten applizierbaren Stoßwellengeneratoren (3, 4), **dadurch gekennzeichnet,** daß die Halterung (7, 8, 9) so ausgebildet ist, daß der Einstrahlwinkel für jeden Stoßwellengenerator (3, 4) individuell einstellbar ist.

2. Lithotripsie-Arbeitsplatz nach Anspruch 1, **dadurch gekennzeichnet,** daß die Halterung (7, 8, 9) eine etwa kreisförmig gekrümmte Führung (7) für die individuelle Führung der Stoßwellengeneratoren (3, 4) aufweist.

3. Lithotripsie-Arbeitsplatz nach Anspruch 2, **dadurch gekennzeichnet,** daß die Führung (7) mittels eines Kugelgelenkes (8) allseitig - schwenkbar gelagert ist.

0 277 489